# EUROPÄISCHE PATENTANMELDUNG

(11) **EP 0 789 018 A1**
(43) Veröffentlichungstag der Anmeldung: **13.08.1997**
(21) Anmeldenummer: 97101412.1
(22) Anmeldetag: 30.01.1997
(51) Int. Cl.: C07D 233/52

(54) **Hydrierung von halonitroaromatischen Verbindungen**

(30) Priorität: 12.02.1996 DE 19604988
(71) Anmelder: BAYER AG, 51368 Leverkusen (DE)
(72) Erfinder: Mais, Franz-Josef, Dr., 40591 Düsseldorf (DE); Paetz, Klaus-Christian, Dr., 51399 Burscheid (DE); Fiege, Helmut, Dr., 51373 Leverkusen (DE); Blank, Heinz Ulrich, Dr., 51519 Odenthal (DE); vor der Brueck, Dieter, Dr., 53113 Bonn (DE); Mehl, Wolf, Dr., 50733 Köln (DE)

(57) **Zusammenfassung**

Verfahren zur Herstellung von haloaromatischen Aminen durch katalytische Hydrierung der entsprechenden halonitroaromatischen Verbindungen, dadurch gekennzeichnet, daß man als Katalysator eisenhaltigen Raney-Nickel einsetzt, wobei die hergestellten haloaromatischen Amine zur Synthese von photographischen Kupplern, nutzbar in photographischen Emulsionen oder Elementen, eingesetzt werden können.

## Beschreibung

Die Erfindung betrifft ein Verfahren zur Herstellung von haloaromatischen Aminen durch katalytische Hydrierung der entsprechenden Nitroverbindungen mit eisenhaltigem Raney-Nickel.

Probleme, die bei der Herstellung von haloaromatischen Aminen durch katalytische Hydrierung aus den entsprechenden Nitroverbindungen auftreten, betreffen beispielsweise die zu schwache Katalysator-Aktivität oder -Selektivität, was zu einer unvollständigen Umsetzung des eingesetzten Eduktes oder zu substantiellen Anteilen an unerwünschten Enthalogenierungsprodukten als Nebenprodukten führt. Verunreinigungen durch Nebenprodukte und gegebenenfalls nicht umgesetztes Edukt machen zusätzliche Reinigungsschritte notwendig, was in der Regel mit enormen zusätzlichem Aufwand verbunden ist.

Hohe Reinheitsanforderungen werden insbesondere an die zur Herstellung von Pharmazeutika und photographischen Chemikalien verwendeten haloaromatischen Amine gestellt.

Im Stand der Technik sind bereits zahlreiche Wege beschritten worden, um haloaromatische Amine unter Erhalt des Halogenatoms aus der entsprechenden Nitroverbindung mittels katalytischer Hydrierung bereitzustellen.

Zur Erreichung dieses Zwecks werden beispielsweise Sulfide von Platin, Rhodium, Ruthenium oder Kobalt (US-A-3.350.450) oder sulfidierte oder sulfitierte Edelmetallkohlekontakte (US-A-3.761.425, US-A-3.929.891) als Katalysator eingesetzt. In WO-A-89/07096 wird die Enthalogenierung derartiger Verbindungen durch Verwendung eines chromhaltigen Raney-Kobalt-Katalysators verhindert, wofür jedoch aufwendige Verfahrensschritte bzw. -parameter zu unbefriedigenden Raum-Zeit-Ausbeuten führen. In DD-A-159 875 werden u.a. haloaromatische Amine durch katalytische Hydrierung der entsprechenden Nitroverbindungen mit Raney-Nickel als Katalysator erhalten. Jedoch ist bei dieser Verfahrensweise der Anteil an Enthalogenierungsprodukten noch sehr hoch.

Es wurde nun ein Verfahren zur Herstellung von haloaromatischen Aminen durch katalytische Hydrierung der entsprechenden halonitroaromatischen Verbindungen gefunden, das dadurch gekennzeichnet ist, daß man als Katalysator eisenhaltiges Raney-Nickel einsetzt.

Unter einem haloaromatischen Amin wird im Rahmen dieser Anmeldung eine Verbindung verstanden, die mindestens eine Aminogruppe an den C-Atomen eines aromatischen carbocyclischen Ringes trägt und mindestens ein Halogenatom an den C-Atomen desselben Ringes oder an den C-Atomen anderer carbocyclischer aromatischer Ringe trägt. Unter halonitroaromatischer Verbindung wird im Rahmen dieser Anmeldung die entsprechende Nitroverbindung verstanden.

Als carbocyclische aromatische Ringe kommen insbesondere gegebenenfalls weiter substituierte Benzol- oder Naphthalinringe in Frage.

Bevorzugte halonitroaromatische Verbindungen sind nitroaromatische Bromide, Iodide sowie Chloride. Besonders bevorzugt sind nitroaromatische Chloride.

Die in dem erfindungsgemäßen Verfahren bevorzugt einzusetzenden halonitroaromatischen Verbindungen bzw. die dabei erhaltenen entsprechenden haloaromatischen Amine entsprechen den Formeln (I) bzw. (II)

A-NO₂ (I)

A-NH₂ (II),

worin
- A: ein gegebenenfalls substituierter aromatischer Rest ist, der an die NO₂-Gruppe in Formel (I) bzw. die NH₂-Gruppe in Formel (II) durch ein Kernkohlenstoffatom eines aromatischen Ringes gebunden ist
und wobei mindestens ein Kernkohlenstoffatom eines aromatischen Ringes des Restes A durch mindestens ein Halogenatom, insbesondere Chlor, substituiert ist.

Der aromatische Rest A kann weitere Substituenten tragen wie beispielsweise Hydroxy, Alkyl, Alkoxy, Aryloxy, Aryl, Formyl, Alkanoyl, Cycloalkanoyl, Aroyl, Alkanoyloxy, Cycloalkanoyloxy, Aroyloxy, Alkylsulfonyl, Cycloalkylsulfonyl, Arylsulfonyl, Alkanoylamino, Cycloalkanoylamino, Aroylamino, Alkylsulfonamido, Cycloalkylsulfonamido, Arylsulfonamido, Alkoxycarbonyl, Cycloalkoxycarbonyl, Aryloxycarbonyl, Carbamoyl, Sulfamoyl, Alkyl-, Aryl- oder Heterocyclyl-aminocarbonyl oder -carbonylamino und substituiertes Amino.

Die Alkylgruppen in der Liste der obengenannten Substituenten können unverzweigte oder verzweigte, unsubstituierte oder substituierte C₁-C₂₀-Alkyl-gruppen sein. Beispiele solcher Alkylgruppen sind: Methyl, Ethyl, Propyl, 2-Methoxypropyl, 1,1-Dimethylpropyl, Hexyl, Octyl, 2-Ethylhexyl, Decyl, Dodecyl, Hexadecyl etc.

Als Cycloalkylgruppen sind unsubstituierte oder substituierte C₅-C₇-Cycloalkyle bevorzugt wie Cyclopentyl, Cyclohexyl, Cycloheptyl usw. Die Arylreste sind vorzugsweise carbocyclische, unsubstituierte oder substituierte C₈-C₁₀-Aromaten, wie Phenyl oder Naphthyl. Carbamoyl- oder Sulfamoyl-Gruppen können unsubstituiert oder substituiert sein, wobei als mögliche Substituenten Alkyl, Cycloalkyl und Aryl zu nennen sind. Die substituierten Aminogruppen können ein oder zwei Alkyl-, Cycloalkyl-, Aryl- oder heterocyclische Reste tragen.

Als besonders bevorzugte Substituenten des aromatischen Restes A sind die nachfolgenden zu nennen:

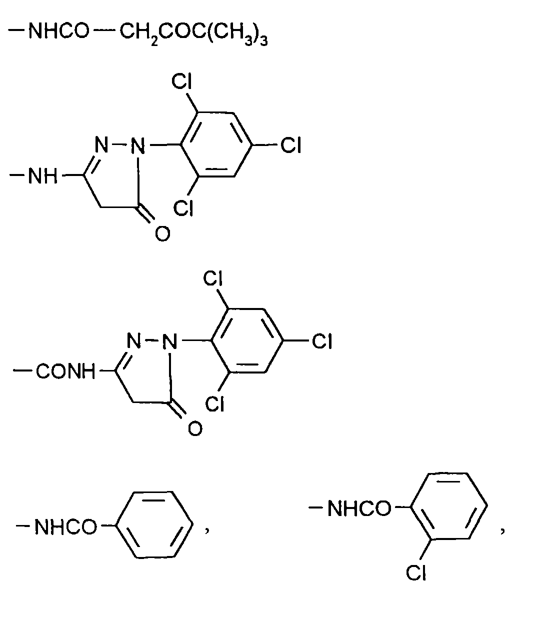

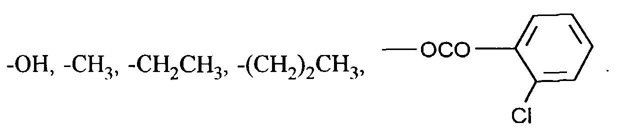

Der aromatische Rest ist vorzugsweise ein gegebenenfalls substituierter Benzol- oder Naphthalinrest, insbesondere ein Benzolrest.

Der aromatische Rest A trägt an dem aromatischen Ring, an dem die NO₂-Gruppe sitzt, neben dem oder den gegebenenfalls vorhandenen Halogenatom(en) vorzugsweise noch ein bis zwei der oben genannten Substituenten. Als aromatischer Rest A mit zwei Halogenatomen ist beispielsweise 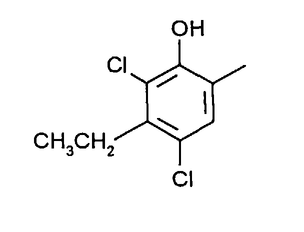 zu nennen.

Bevorzugte halonitroaromatische Verbindungen der Formel I entsprechen den Formeln Ia, Ib oder Ic und bevorzugte haloaromatische Amine der Formel II entsprechen den Formeln IIa, IIb oder IIc, 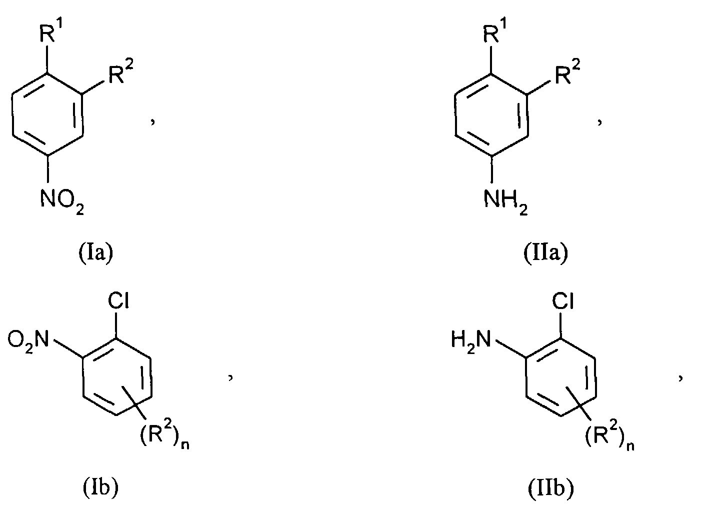 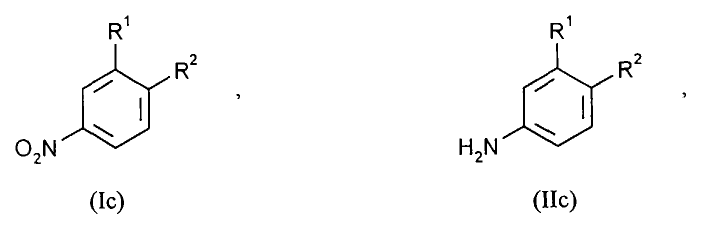 worin
- R¹: für Wasserstoff oder Chlor steht,
- n: für 1 oder 2 steht und
- R²: die Bedeutung der möglichen Substituenten für A annehmen kann, wobei für n = 2 R² auch unterschiedliche Bedeutungen besitzen kann.

Besonders bevorzugte Reste für R² sind

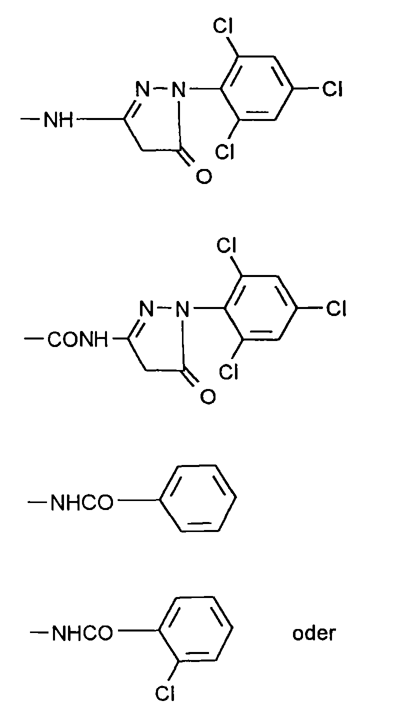

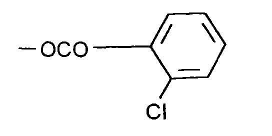

Besonders bevorzugte halonitroaromatische Verbindungen entsprechen einer Verbindung der Formeln (Id) bis (Ig) und die entsprechenden haloaromatischen Amine entsprechen einer Verbindung der Formeln (IId) bis (IIg): 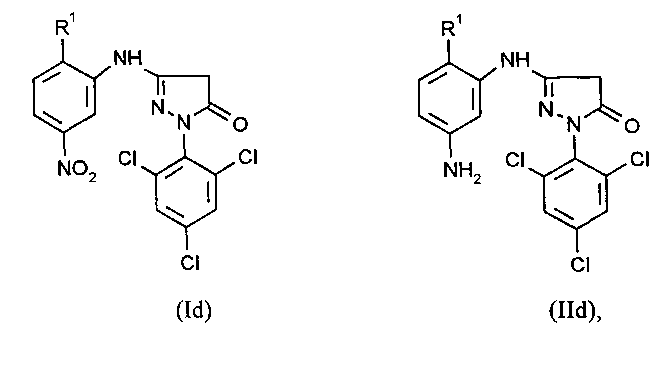 worin
- R¹: Wasserstoff oder insbesondere Chlor bedeutet,

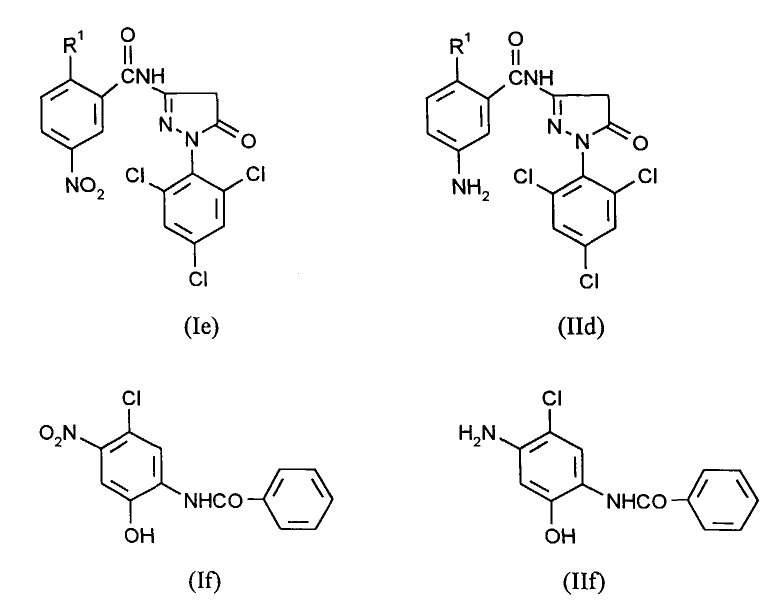

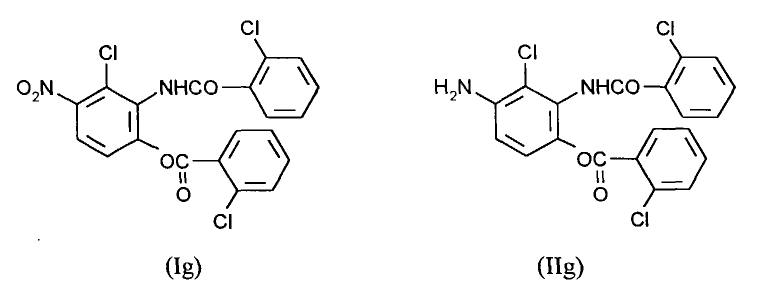

Als Katalysatoren werden erfindungsgemäß eisenhaltige Raney-Nickelkatalysatoren eingesetzt. Diese Katalysatoren können nach den üblichen Methoden zur Präparation von Raney-Katalysatoren hergestellt werden (H. Adkins et. al. Org. Syntheses, Coll. Vol. III, 180 (1955)). Üblicherweise stellt man eine Legierung aus ca. 50 Gew.-% Aluminium und verschiedenen Anteilen an Nickel und Eisen her. Aus der zerkleinerten Legierung wird der größte Teil des Aluminiums durch wäßrige Natronlauge in der Hitze ausgelaugt und so der Katalysator aktiviert. Erfindungsgemäß enthalten die aktivierten Katalysatoren 1 bis 10 %, bevorzugt 3 bis 8 % Restaluminium, 5 bis 30 %, bevorzugt 10 bis 20 % Eisen und den Rest an Nickel (als Gehalte in der Trockenmasse). Die Menge der einzusetzenden Katalysatoren ist im Prinzip unkritisch. Zu geringe Mengen ergeben jedoch zu lange Hydrierzeiten während zu große Mengen unwirtschaftlich sind. Erfindungsgemäß setzt man beispielsweise 0,1 bis 20 Gew.-% an Katalysatortrockenmasse, bezogen auf halonitroaromatische Verbindung, bevorzugt 0,5 bis 15 Gew.-%, besonders bevorzugt 1 bis 10 Gew.-% ein.

Vorzugsweise ist von dem erfindungsgemäßen Verfahren die Herstellung von 2-Amino-6-Chlorphenyl-alkylsulfanen aus den entsprechenden Nitroverbindungen ausgenommen, sofern sie durch katalytische Hydrierung ohne Zusatz einer weiteren Schwefelverbindung in Gegenwart eines Lösungsmittels erfolgt.

Das erfindungsgemäße Verfahren wird im allgemeinen in einem organischen Lösungsmittel durchgeführt. Als organische Lösungsmittel sind beispielsweise aliphatische mit Wasser mischbare Alkohole mit 1 bis 4 C-Atomen wie Methanol, Ethanol, 2-Methoxyethanol oder iso-Propanol zu nennen. Besonders bevorzugt ist Methanol.

Das erfindungsgemäße Verfahren kann auch in einem Gemisch aus organischem Lösungsmittel und Wasser durchgeführt werden. Dabei ist es vorteilhaft wenn der Wassergehalt kleiner als 50 Gew.-%, bevorzugt kleiner als 10 Gew.-% ist.

Das organische Lösungsmittel kann auch im Gemisch mit weiteren Co-Solventien eingesetzt werden. Solche Co-Solventien sind beispielsweise Dimethylformamid, Dimethylacetamid sowie Tetrahydrofuran.

Auch andere Lösungsmittel können verwendet werden. Zu nennen sind beispielsweise Ester wie Methyl- oder Ethylacetat oder Ether wie Tetrahydrofuran oder Diisopropylether.

Die Löslichkeit schwer löslicher halonitroaromatischer Verbindungen, die ein acides Wasserstoffatom tragen, kann dadurch verbessert werden, daß man der Reaktion eine Base zugibt. Bevorzugt ist der Zusatz einer anorganischen Base wie Alkalihydroxid oder Alkalicarbonat. Bevorzugt ist der Zusatz von Alkalihydroxid. Der Zusatz von Alkalihydroxid kann als wäßrige Lösung erfolgen. Eine weitere bevorzugte Gruppe von Basen sind die Alkalialkoholate von niederen aliphatischen Alkoholen gegebenenfalls als Lösung in dem entsprechenden Alkohol.

Für die Durchführung des erfindungsgemäßen Verfahrens ist es jedoch nicht zwingend erforderlich, daß die gesamte halonitroaromatische Verbindung vollständig gelöst ist. Dementsprechend ist die Menge der gegebenenfalls zuzusetzenden Basen in den obengenannten Fällen in weiten Grenzen variabel. Besonders vorteilhaft ist eine Basenmenge von 50 bis 120 mol-%, bezogen auf die halonitroaromatische Verbindung, bevorzugt von 75 bis 110 mol-%, besonders bevorzugt von 90 bis 110 mol-% anzuwenden.

Erfindungsgemäß ist es möglich, eine Überdosierung von Base durch den Zusatz geringer Mengen Säure abzustumpfen. Als Säuren kommen beispielsweise einfache Mineralsäuren wie HCl, H₂SO₄ oder H₃PO₄ oder einfache Carbonsäuren wie Essigsäure, Propionsäure usw. in Frage. Bevorzugt sind einfache Carbonsäuren und Phosphorsäure.

Die Menge an zuzusetzender Säure ist entsprechend der Überdosierung der Base so zu wählen, daß die verbleibende Basemenge in einem Bereich von 50 bis 120 mol-%, bezogen auf die halogenaromatische Verbindung, bevorzugt 75 bis 110 mol-%, besonders bevorzugt 90 bis 110 mol-% liegt.

Der Einsatz von Basen wie oben beschrieben ist besonders bevorzugt wenn halonitroaromatische Verbindungen der Formel (Id) bis (Ig) eingesetzt werden.

Das erfindungsgemäße Verfahren wird vorzugsweise bei geringem Überdruck an Wasserstoff durchgeführt. Vorteilhaft ist ein Bereich von 0,1 bis 30 bar, bevorzugt 0,5 bis 15 bar H₂ Überdruck.

Das erfindungsgemäße Verfahren wird in der Regel bei Raumtemperatur oder bei nur leicht erhöhter Temperatur durchgeführt. Vorteilhaft ist ein Bereich von 20 bis 80°C, bevorzugt 25 bis 60°C, besonders bevorzugt 25 bis 45°C.

Bevorzugt ist es weiterhin, das erfindungsgemäße Verfahren einstufig d.h. ohne Unterbrechung zur Zwischendosierung von Reagenzien durchzuführen.

Das erfindungsgemäße Verfahren kann kontinuierlich, halbkontinuierlich oder diskontinuierlich durchgeführt werden. Eine beispielhafte Ausführungsform ist die folgende: Man legt in einem Autoklaven die halonitroaromatische Verbindung, das Lösungsmittel und gegebenenfalls die Base vor, gibt nach Verrühren den eisenhaltigen Raney-Nickel Katalysator zu und drückt Wasserstoff beispielsweise 5-10 bar auf. In exothermer Reaktion hydriert man bis zur Druckkonstanz, belüftet, filtriert den Katalysator ab und isoliert das Produkt vorzugsweise durch Fällen mit wäßriger Säure.

An dem erfindungsgemäßen Verfahren ist ausgesprochen überraschend, daß der Einsatz des eisenhaltigen Raney-Nickel Katalysators eine derart starke Verminderung der Enthalogenierung im Vergleich zum Stand der Technik (Raney-Ni) bewirkt.

Die nach dem erfindungsgemäßen Verfahren hergestellten Amine, vorzugsweise die der Formel II, werden zur Herstellung von photographischen Kupplern verwendet. Dazu wird das Amin mit einem Säurechlorid zu einem Amid umgesetzt. Zahlreiche Beispiele sind dafür in der Literatur bekannt (siehe z.B. US-A- 4 443 536 und die dort in Spalte 1, Zeile 36 bis Spalte 2, Zeile 18 zitierte Literatur).

Als bevorzugte Säurechloride können beispielsweise 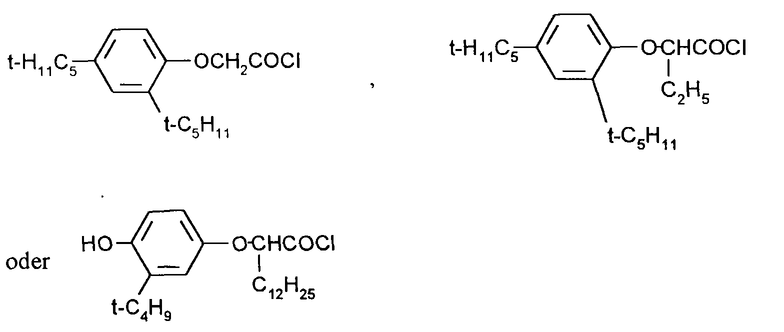 genannt werden.

Wichtige photografische Kuppler, die aus den erfindungsgemäß hergestellten Aminen der Formel II hergestellt werden können, sind beispielsweise die folgenden:

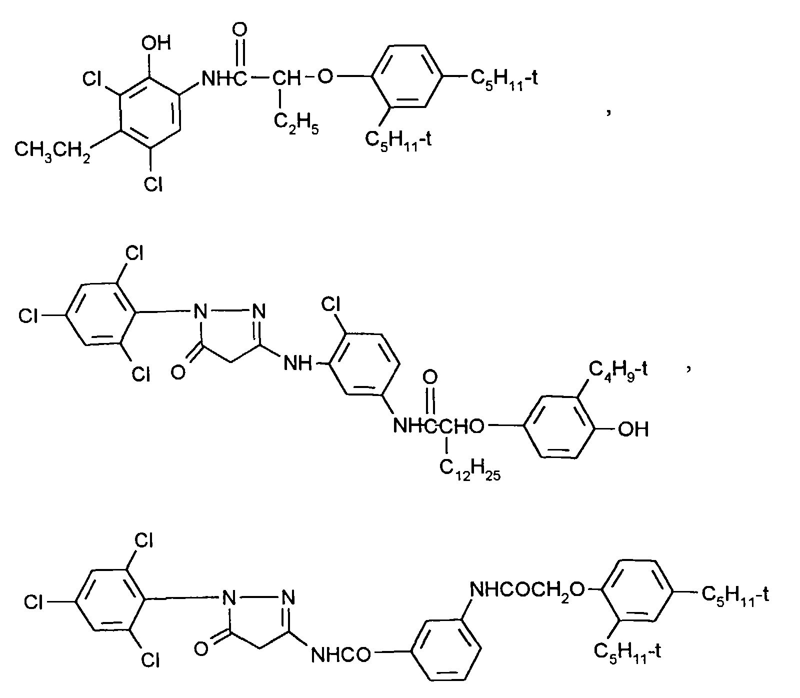

Diese Kuppler werden vorzugsweise in photografischen Emulsionen oder Elementen eingesetzt.

Die nachfolgenden Beispiele erläutern die Erfindung, ohne sie jedoch darauf einzuschränken.

### Beispiel 1

In einem Stahlautoklaven wurden 100 Gew.-Teile an 3-(5-Nitro-2-chlorophenylamino)-1-(2,4,6-trichlorphenyl)-2-pyrazolin-5-on der Formel (Ib), worin R³ für Cl steht (Gehalt laut HPLC: 95 %), 366 Gew.-Teile technisches Methanol, 17,5 Gew.-Teile 50 % wäßrige Natronlauge und 6,2 Gew.-Teile 50 % wäßriges Raney-Nickel/Eisen der Zusammensetzung 80,5 % Nickel, 14,2 % Eisen und 5,0 % Aluminium vorgelegt und nach Inertisieren mit Stickstoff mit 5 bar H₂ bei Umgebungstemperatur unter Rühren begast. Es setzte eine exotherme Reaktion ein. Durch Kühlung wurde die Temperatur bei 30°C gehalten. Nach 7 Stunden wurde entspannt, mit Stickstoff inertisiert, der Katalysator durch Filtration abgetrennt und die klare Reaktionslösung bei 25 bis 30°C in eine Mischung aus 835 Gew.-Teilen Wasser und 20,2 Gew.-Teilen Essigsäure eingetragen. Die entstandene Suspension wurde auf 0°C unter Rühren gekühlt, und filtriert. Der zurückbleibende Feststoff wurde mit 800 Gew.-Teilen Wasser gewaschen, trockengesaugt und im Vakuum bei 50°C getrocknet. Man erhielt 91,0 g eines sehr hellbeigen feinen Pulvers. Die HPLC-Analyse ergab einen Gehalt von 99,7 %. Entchlorierte Verbindungen wurden im HPLC nicht gefunden (d.h. Gehalt <0,1 %). Somit betrug die Ausbeute 98,5 % der Theorie.

### Beispiel 2

In einem Stahlautoklaven wurden 100 Gew.-Teile an 3-(5-Nitro-2-chlorophenylamino)-1-(2,4,6-trichlorphenyl)-2-pyrazolin-5-on der Formel (Ib), worin R³ = Cl (Gehalt laut HPLC 95 %), 314 Gew.-Teile Methanol, 19,0 Gew.-Teile 50 % wäßrige Natronlauge, 1,4 Gew.-Teile Essigsäure und 10,7 Gew.-Teile 50 % wäßriges Raney-Nickel/Eisen der gleichen Zusammensetzung wie im Beispiel 1 vorgelegt. Nach Inertisieren mit Stickstoff wurde mit 5 bar H₂ unter Rühren begast. Man hielt die Temperatur 4 Stunden bei maximal 30°C, entspannte, inertisierte mit Stickstoff, Filtrierte den Katalysator ab und gab die filtrierte Lösung in eine Mischung aus 716 Gew.-Teilen Wasser und 17,3 Gew.-Teilen Essigsäure. Nach dem Abkühlen auf 0°C isolierte man den Feststoff durch Filtrieren und Waschen mit 680 Gew.-Teilen Wasser. Nach dem Trocknen erhielt man 105,7 g eines fast farblosen feinen Pulvers. Die HPLC-Analyse analog Beispiel 1 ergab einen Gehalt von 100,0 %. Entchlorierte Verbindungen wurden nicht gefunden. Die Ausbeute betrug somit 98,4 % der Theorie.

### Beispiel 3

In einem Stahlautoklaven wurden 100 Gew.-Teile an 3-(5-Nitro-2-chlorophenylamino)-1-(2,4,6-trichlorphenyl)-2-pyrazolin-5-on der Formel (Ib), worin R³ = Cl einer weniger guten Qualität (Gehalt laut HPLC: 76 %) 352 Gew.-Teile Methanol, 17,4 Gew.-Teile 50 % wäßrige Natronlauge, 1,4 Gew.-Teile Essigsäure und 12,0 Gew.-Teile 50 % wäßriges Raney-Nickel/Eisen der Zusammensetzung, 80,3 % Nickel, 13,7 % Eisen und 5,7 % Aluminium vorgelegt. Nach dem Inertisieren mit Stickstoff wurde mit 5 bar H₂ unter Rühren begast. Man hielt die Temperatur 2 h 45 min bei max. 30°C, entspannte, inertisierte mit Stickstoff, filtrierte den Katalysator ab, und ließ die klare Lösung bei Raumtemperatur in eine Mischung aus 800 Gew.-Teilen Wasser und 15,9 Gew.-Teilen Essigsäure einlaufen. Die so erhaltene Suspension wurde unter Rühren auf 0°C abgekühlt. Der Feststoff wurde abgesaugt, mit 830 Gew.-Teilen Wasser gewaschen und im Vakuum bei 50°C getrocknet. Man erhielt 76,5 g eines nahezu farblosen feinen Pulvers. Die HPLC-Analyse analog Beispiel 1 ergab einen Gehalt von 99,9 %. Entchlorierte Verbindungen wurden nicht nachgewiesen. Die Ausbeute betrug somit 99,7 % der Theorie.

### Beispiel 4

In einem Stahlautoklaven wurden 100 Gew.-Teile an 3-(5-Nitro-2-chlorophenylamino)-1-(2,4,6-trichlorphenyl)-2-pyrazolin-5-on der Formel (Ib), worin R³ = Cl (Gehalt laut HPLC: 92 %), 440 Gew.-Teile Methanol, 19,0 Gew.-Teile 50 % wäßrige Natronlauge, 1,4 Gew.-Teile Essigsäure und 15,0 Gew.-Teile des 50 % wäßrigen Raney-Nickel/Eisens der gleichen Zusammensetzung wie in Beispiel 3 vorgelegt, nach dem Inertisieren mit Stickstoff wurde mit 10 bar H₂ bei max. 32°C begast. Nach 2 Stunden wurde entspannt, mit Stickstoff inertisiert und der Katalysator abfiltriert. Die klare Lösung wurde in eine Mischung aus 870 Gew.-Teile Wasser und 19,8 Gew.-Teile Essigssäure gegeben. Nach dem Abkühlen der Suspension saugte man den Feststoff ab, wusch mit 800 Gew.-Teilen Wasser und trocknete im Vakuum bei 50°C. Man erhielt 73,6 g eines sehr hellbeigen Pulvers. Die HPLC-Analyse analog Beispiel 1 ergab einen Gehalt von 99,4 %. Als entchloriertes Produkt wurde 0,3 % der Verbindung der Formel 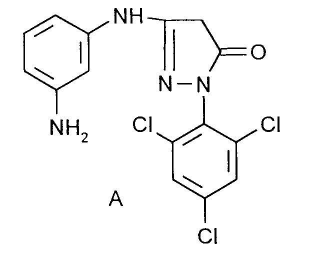 gefunden.

Andere entchlorierte Verbindungen wurden nicht nachgewiesen. Das ergab eine Ausbeute von 98,3 % der Theorie.

Bei sonst gleicher Umsetzung jedoch unter Verwendung eines eisenfreien Raney-Nickel-Katalysators analog DD-A 159 875, Bsp. 5 wurden nur 73,1 g eines beigen feinen Pulvers erhalten, das sehr viel mehr enthalogenierte Produkte enthielt (siehe Tabelle).

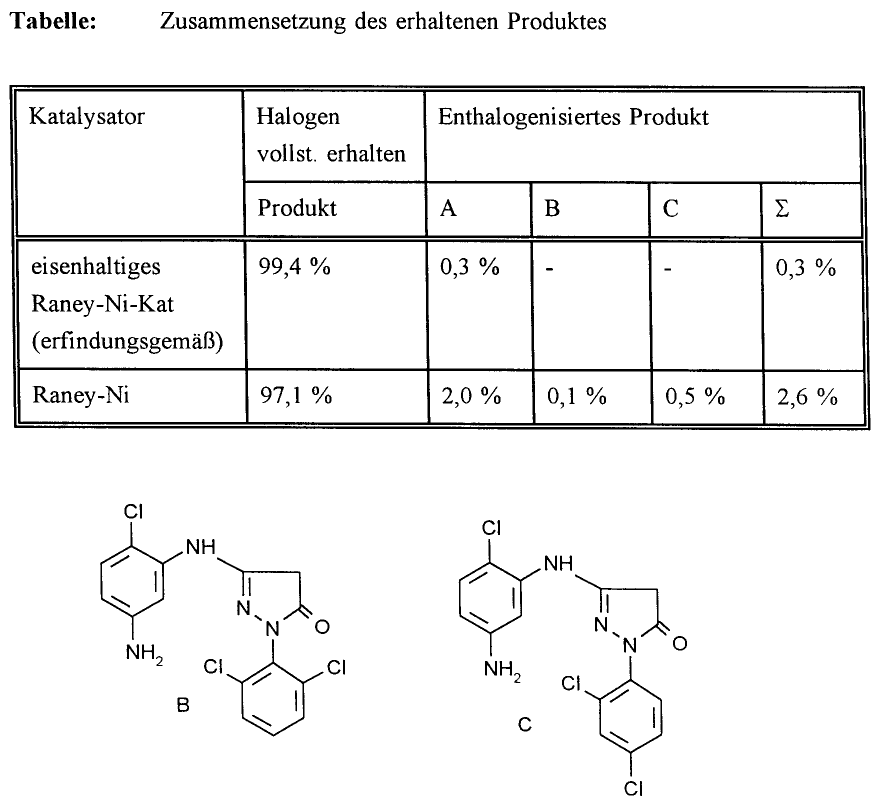

## Patentansprüche

1. Verfahren zur Herstellung von haloaromatischen Aminen durch katalytische Hydrierung der entsprechenden halonitroaromatischen Verbindungen, dadurch gekennzeichnet, daß man als Katalysator eisenhaltiges Raney-Nickel einsetzt.

2. Verfahren gemäß Anspruch 1, dadurch gekennzeichnet, daß die halonitroaromatische Verbindung der Formel (I) und das entsprechende haloaromatische Amin der Formel (II) entsprechen
A-NO₂ (I)
A-NH₂ (II),
worin
A ein gegebenenfalls substituierter aromatischer Rest ist, der an die NO₂-Gruppe in Formel (I) bzw. die NH₂-Gruppe in Formel (II) durch ein Kernkohlenstoffatom eines aromatischen Ringes gebunden ist
und wobei mindestens ein Kernkohlenstoffatom eines aromatischen Ringes des Restes A durch mindestens ein Halogenatom substituiert ist.

3. Verfahren gemäß Anspruch 1, dadurch gekennzeichnet, daß der aromatische Rest A einen Substituenten aus folgender Reihe trägt

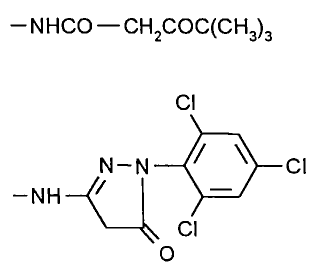

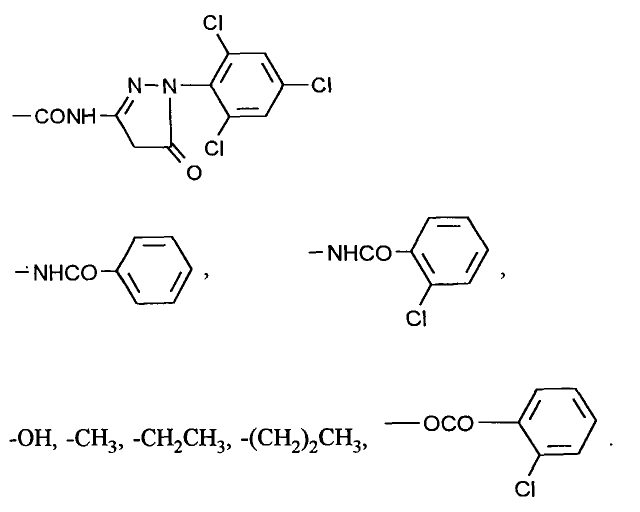

4. Verfahren gemäß Anspruch 1, dadurch gekennzeichnet, daß die halonitroaromatische Verbindung der Formel (I) den Formeln Ia, Ib oder Ic und bevorzugte haloaromatische Amine der Formel II den Formeln IIa, IIb oder IIc, entsprechen 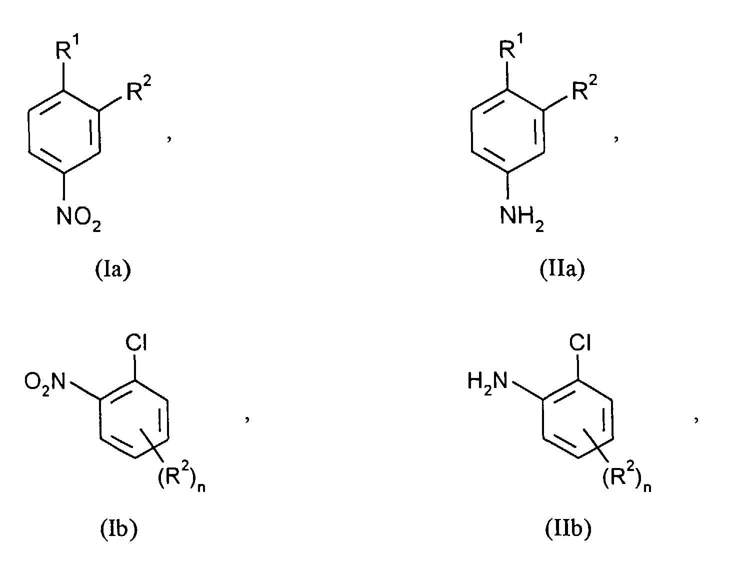 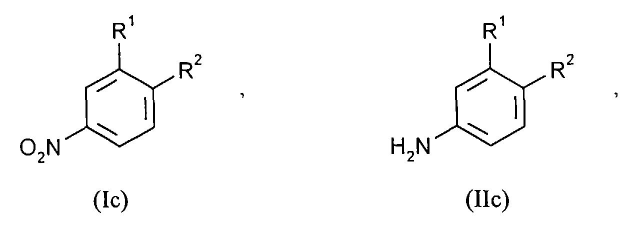 worin
R¹ für Wasserstoff oder Chlor steht,
n für 1 oder 2 steht und
R² Hydroxy, Alkyl, Alkoxy, Aryloxy, Aryl, Formyl, Alkanoyl, Cycloalkanoyl, Aroyl, Alkanoyloxy, Cycloalkanoyloxy, Aroyloxy, Alkylsulfonyl, Cycloalkylsulfonyl, Arylsulfonyl, Alkanoylamino, Cycloalkanoylamino, Aroylamino, Alkylsulfonamido, Cycloalkylsulfonamido, Arylsulfonamido, Alkoxycarbonyl, Cycloalkoxycarbonyl, Aryloxycarbonyl, Carbamoyl, Sulfamoyl, Alkyl-, Aryl- oder Heterocyclyl-aminocarbonyl oder -carbonylamino und substituiertes Amino bedeutet.

5. Verfahren gemäß Anspruch 4, dadurch gekennzeichnet, daß R² für einen der folgenden Reste steht:

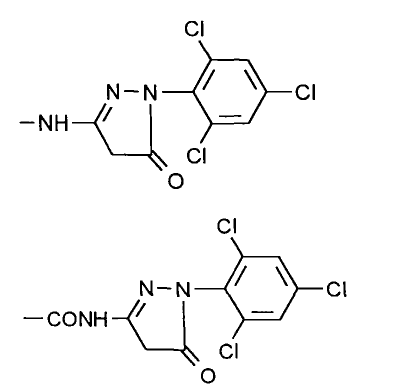

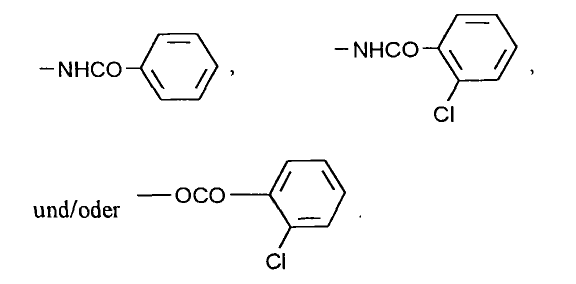

6. Verfahren gemäß Anspruch 1, dadurch gekennzeichnet, daß die halonitroaromatische Verbindung einer der Formeln (Id) bis (Ig) und das haloaromatische Amin einer der Formeln (IId) bis (IIg) entsprechen 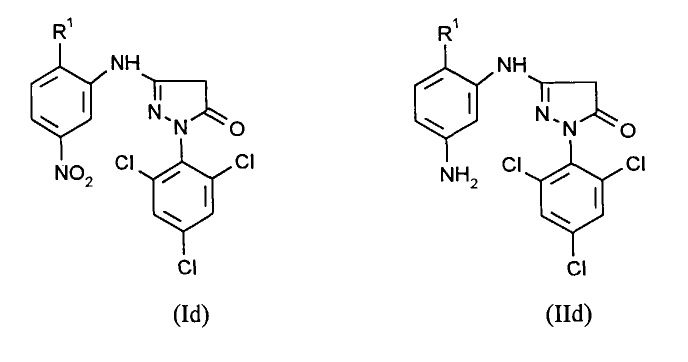 worin
R¹ Wasserstoff oder insbesondere Chlor bedeutet,

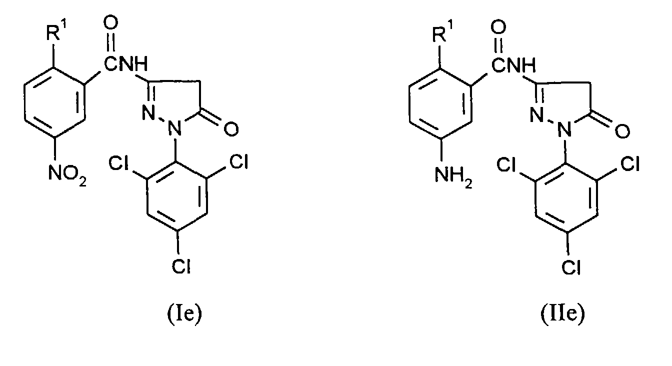

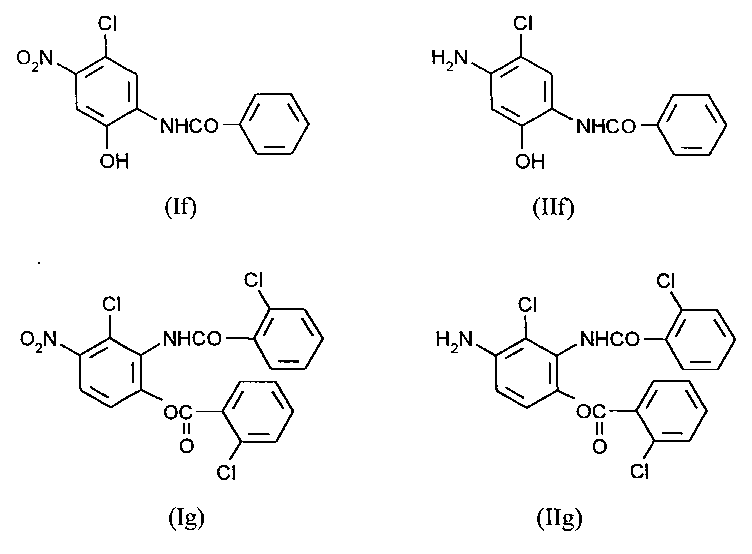

7. Verfahren gemäß Anspruch 1, dadurch gekennzeichnet, daß man die katalytische Hydrierung in einem aliphatischen mit Wasser mischbaren Alkohol mit 1 bis 4 C-Atomen durchführt.

8. Verfahren gemäß Anspruch 1, dadurch gekennzeichnet, daß ein eisenhaltiger Raney-Nickel-Katalysator mit einem Gehalt von 1 bis 10 % Aluminium und 5 bis 30 % Eisen eingesetzt wird.

9. Verwendung eines gemäß einem der Ansprüche 1 bis 8 hergestellten haloaromatischen Amins zur Herstellung eines Farbküpplers, dadurch gekennzeichnet, das man das Amin mit einem Säurechlorid umsetzt.

10. Verwendung gemäß Anspruch 9, dadurch gekennzeichnet, daß als Säurechlorid 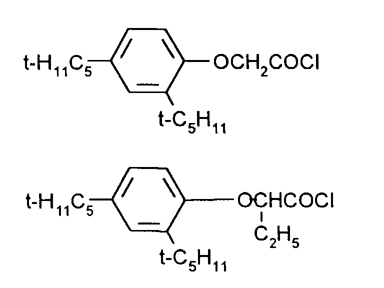 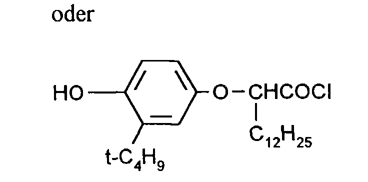 eingesetzt wird.
